(19) Europäisches Patentamt
European Patent Office
Office européen des brevets

(11) Publication number: **0 267 439**
**A2**

(12) # EUROPEAN PATENT APPLICATION

(21) Application number: **87114841.7**

(51) Int. Cl.⁴: **C07D 213/30** , A61K 31/44

(22) Date of filing: **12.10.87**

Claims for the following Contracting States: GR + ES.

(30) Priority: **14.10.86 US 918203**

(43) Date of publication of application:
**18.05.88 Bulletin 88/20**

(84) Designated Contracting States:
**AT BE CH DE ES FR GB GR IT LI NL SE**

(71) Applicant: **G.D. Searle & Co.**
**P.O. Box 5110**
**Chicago Illinois 60680(US)**

(72) Inventor: **Lai, Steve Mun Fook**
**179 River Valley Road**
**Singapore(SG)**
Inventor: **Manley, Paul William**
**Hermann-Suter-Strasse 9**
**CH-4053 Basle(CH)**
Inventor: **Porter, Roderick Alan**
**2 The Ferns Finings Road**
**Lane End High Wycombe(GB)**

(74) Representative: **Beil, Hans Christoph, Dr. et al**
**Beil, Wolff und Beil Rechtsanwälte**
**Adelonstrasse 58**
**D-6230 Frankfurt am Main 80(DE)**

(54) **Alpha-(phenylalkyl) pyridinealkanol derivatives.**

(57) This invention relates to novel α-(phenylalkyl) pyridinealkanol derivatives useful in the treatment of diseases or disorders mediated by platelet-activating factor. This invention further relates to pharmaceutical compositions of such α-(phenylalkyl)pyridinealkanol derivatives.

EP 0 267 439 A2

# α-(PHENYLALKYL)PYRIDINEALKANOL DERIVATIVES

## BACKGROUND OF THE INVENTION

Platelet-activating factor (PAF) has been associated with various biologic activities and pathways, thus making it an important mediator responsible for a variety of physiologic processes including, but not limited to, activation or coagulation of platelets, smooth muscle contraction, pathogenesis of immune complex deposition, inflammation, endotoxin shock, respiratory, cardiovascular and intravascular alterations. These physiological processes are associated with a large group of diseases such as, for example, cardiovascular disorders, asthma, lung edema, adult respiratory distress syndrome and inflammatory diseases.

European Patent Application Nos. 142,333 and 142,801 disclose a class of gylcerol derivatives and indene derivatives respectively, which are used as PAF-antagonists.

## SUMMARY OF THE INVENTION

The present invention relate to a novel class of compounds of the formula:

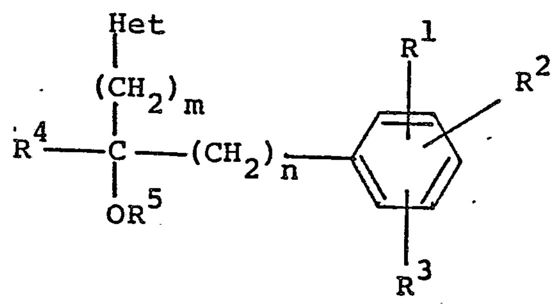

I

wherein $R^1$, $R^2$ and $R^3$ are independently hydrogen, $C_1$-$C_8$ alkoxy, or $C_1$-$C_8$ alkylthio;

$R^4$ is hydrogen or $C_1$-$C_8$ alkyl;

$R^5$ is hydrogen, $-\overset{\text{O}}{\overset{\|}{\text{C}}}-R^6$ or $C_1$-$C_8$ alkyl, wherein $R^6$ is $C_1$-$C_8$ alkyl;

n and m are independently integers of from 1 to 5;

and Het is pyridyl; or a pharmaceutically acceptable acid addition salt thereof.

The invention further relates to pharmaceutical compositions comprising a compound of formula (I). Such compounds and compositions have potent and specific PAF antagonistic activities and are thereby useful in the treatment of various diseases or disorders mediated by PAF, for example, endotoxin shock, inflammation, cardiovascular disorder, asthma, lung edema, and adult respiratory distress syndrome.

## DESCRIPTION

As used herein the term "$C_1$-$C_8$ alkyl" refers to straight chain or branched chain hydrocarbon groups having from one to eight carbon atoms. Illustrative of such alkyl groups are methyl, ethyl, propyl, isopropyl, butyl, isobutyl, sec-butyl, tert-butyl, pentyl, neopentyl, hexyl, isohexyl, heptyl, octyl and the like.

As used herein the term "$C_1$-$C_8$ alkoxy" refers to straight chain or branched chain alkoxy groups having from one to eight carbons. Illustrative of such alkoxy groups are methoxy, ethoxy, propoxy, isopropoxy, butoxy, tert-butoxy, pentoxy, hexoxy, heptoxy, octoxy and the like.

As used herein the term "$C_1$-$C_8$ alkylthio" refers to straight chain or branched chain alkylthio groups having from one to eight carbon atoms. Illustrative of such alkylthio groups are methylthio, ethylthio, propylthio, isopropylthio, butylthio, isobutylthio, tert-butylthio, pentylthio, hexylthio, heptylthio, octylthio and the like.

The term "pharmaceutically acceptable acid addition salt" refers to a salt prepared by contacting a

compound of formula (I) with an acid whose anion is generally considered suitable for human consumption. Examples of pharmacologically acceptable acid addition salts include the hydrochloride, hydrobromide, hydroiodide, sulfate, phosphate, acetate, propionate, lactate, maleate, malate, succinate, and tartrate salts.

The preferred compounds of the present invention include compounds of the formula

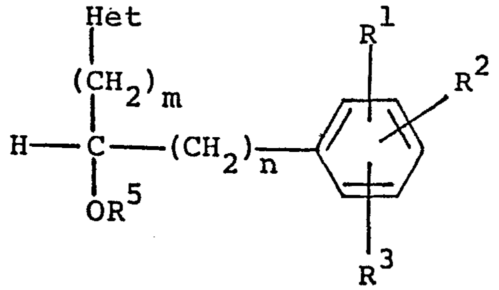

II

wherein m is 1 or 2, n is from 2 to 4, $R^5$ is hydrogen or $C_1$-$C_6$ alkyl; $R^1$, $R^2$ and $R^3$ are independently $C_1$-$C_4$ alkoxy; and Het is 3-pyridyl. A more preferred embodiment includes compounds of formula (II) wherein $R^5$ is hydrogen or methyl, m is 1, n is 3, and $R^1$, $R^2$ and $R^3$ are independently hydrogen or methoxy, and Het is 3-pyridyl.

In addition, certain novel intermediates of the formula

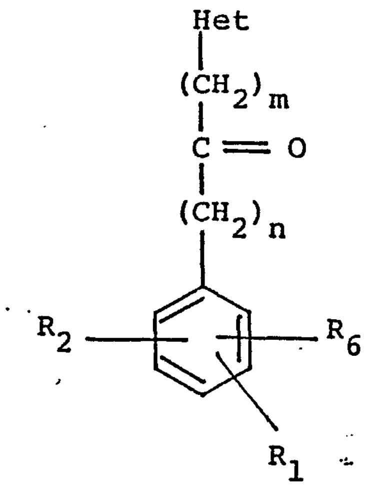

are active as PAF antagonists.

This invention also relates to a method of treatment for patients (or mammalian animals raised in the dairy, meat, or fur industries or as pets) suffering from disorders or diseases which can be attributed to PAF as previously described, and more specifically, a method of treatment involving the administration of compound (I) as the active ingredient.

Accordingly, compound (I) can be used among other things to reduce inflammation, to correct respiratory, cardiovascular, and intravascular alterations or disorders, and to regulate the activation or coagulation of platelets, the pathogenesis of immune complex deposition and smooth muscle contractions.

For the treatment of inflammation, cardiovascular disorders, asthma, or other diseases mediated by PAF, compound (I) may be administered orally, topically, parenterally, or by inhalation spray or rectally in dosage unit formulations containing conventional non-toxic pharmaceutically acceptable carriers, adjuvants and vehicles. The term parenteral as used herein includes subcutaneous, intravenous, intramuscular, or intrasternal injection or infusion techniques.

The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art.

Accordingly, the invention provides a class of novel pharmaceutical compositions comprising one or more compounds of the present invention in association with one or more non-toxic, pharmaceutically acceptable carriers and/or diluents and/or adjuvants (collectively referred to herein as "carrier" materials)

and if desired other active ingredients. The compounds and composition may for example be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit contained in a particular amount of the active ingredient. Examples of such dosage units are tablets or capsules. These may with advantage contain an amount of active ingredient from about 1 to 250 mg preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight may be appropriate.

The active ingredient may also be administered by injection as a composition wherein, for example, saline, dextrose or water may be used as a suitable carrier. A suitable daily dose is from about 0.1 to 100 mg per kg body weight injected per day in multiple doses depending on the disease being treated. A preferred daily dose would be from about 1 to 30 mg/kg body weight.

The dosage regimen for treating an infectious disease condition with the compounds and/or compositions of this invention is selected in accordance with a variety of factors, including the type, age, weight, sex and medical condition of the patient; the severity of the infection; the route of administration; and the particular compound employed and thus may vary widely.

For therapeutic purposes, the compounds of this invention are ordinarily combined with one or more adjuvants appropriate to the indicated route of administration. If per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulphuric acids, gelatin, acacia, sodium alginate, polyvinylpyrrolidone, and/or polyvinyl alcohol, and thus tableted or encapsulated for convenient administration. Alternatively, the compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art. Appropriate dosages, in any given instance, of course depend upon the nature and severity of the condition treated, the route of administration, and the species of mammal involved, including its size and any individual idiosyncrasies.

Representative carriers, diluents and adjuvants include for example, water, lactose, gelatin, starches, magnesium stearate, talc, vegetable oils, gums, polyalkylene glycols, petroleum jelly, etc. The pharmaceutical compositions may be made up in a solid form such as granules, powders or suppositories or in a liquid form such as solutions, suspensions or emulsions. The pharmaceutical compositions may be subjected to conventional pharmaceutical operations such as sterilization and/or may contain conventional pharmaceutical adjuvants such as preservatives, stabilizers, wetting agents, emulsifiers, buffers, etc.

Dosage levels of the order from about 1 mg to about 100 mg per kilogram of body weight per day are useful in the treatment of the above-indicated conditions (from about 50 mg to about 5 mgs. per patient per day). For example, inflammation is effectively treated and anti-pyretic and analgesic activity manifested by the administration from about 25 to about 75 mg of the compound per kilogram of body weight per day (about 75 mg to about 3.75 gms per patient per day). Preferably, from about 5 mg to about 50 mg per kilogram of body weight per daily dosage produces highly effective results (about 250 mg to about 2.5 gm per patient per day).

The amount of active ingredient that may be combined with the carrier materials to produce a single dosage form will vary depending upon the host treated and the particular mode of administration. For example, a formulation intended for the oral administration of humans may contain from 5 mg to 95 mg of active agent compounded with an appropriate and convenient amount of carrier material which may vary from about 5 to 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following Examples are intended to further illustrate the present invention and not to limit the invention in spirit or scope. In the Examples, all parts are parts by weight unless otherwise expressly set forth.

EXAMPLE 1

α-[3-(4-Methoxyphenyl)propyl]-3-pyridineethanol

a) 5-(4-Methoxyphenyl)-1-pentene

To a solution containing 4-methoxyphenethyl magnesium iodide (prepared from the reaction of 4-methoxyphenethyl iodide (60g, 0.23mol) and excess magnesium turnings) in dry tetrahydrofuran (200 ml) at 0°C under a nitrogen atmosphere was added allyl bromide (46.7g, 0.33 mol). The resulting mixture was stirred at room temperature for 14 hours. The reaction mixture was washed with saturated aqueous ammonium chloride solution (3 × 100 ml) and the aqueous washings were back extracted with dichloromethane (3 × 100 ml). The tetrahydrofuran and dichloromethane solutions were combined, washed with aqueous sodium thiosulfate solution, dried over anhydrous magnesium sulfate and concentrated under reduced pressure to yield an oil which was distilled to yield 5-(4-methoxyphenyl)-1-pentene as a pale yellow oil (b.p. 65°C, 0.3 mm. Hg) having the following physical characteristics:

$^1$H-NMR (δ-$CDCl_3$): 1.68 (m,2H), 2.07 (m,2H), 2.55 (m,2H), 3.70 (s,3H), 4.90-5.10 (m,2H), 5.70-5.90 (m,1H), 6.81 and 7.06 (ABq,4H).

b) 3-(4-Methoxyphenyl)propyloxirane

A mixture of 5-(4-methoxyphenyl)-1-pentene (18.9 g, 0.107 mol) and 3-chloroperoxybenzoic acid (22.3 g of 85%, 0.11 mol) in dichloromethane (250 ml) was stirred at room temperature for 25 minutes. Dichloromethane (150 ml) was added to the reaction mixture and the resulting solution was washed with a saturated aqueous sodium hydrogen carbonate solution (3 × 300 ml), dried over anhydrous magnesium sulfate and concentrated to yield an oil which was purified by column chromatography (silica gel, chloroform) to yield 3-(4-methoxyphenyl)propyloxirane as a colorless oil having the following physical characteristics:

$^1$H-NMR (δ-$CDCl_3$): 1.40-1.80 (m,4H), 2.40 (m,1H), 2.58 (t,2H), 2.68 (m,1H), 2.86 (m,1H), 3.70 (s,3H), 6.81 and 7.06 (ABq,4H).

c) α-[3-(4-Methoxyphenyl)propyl]-3-pyridineethanol

A solution of 3-bromopyridine (3.15 g, 0.020 mol) in dry ether (10 ml) was added slowly over a period of 1 hour to a stirred solution of n-butyl lithium (15 ml of 1.4 M in hexane, 0.021 mol) in dry ether (10 ml), at a temperature of -78°C and under a nitrogen atmosphere. The resulting yellow suspension was stirred at -78°C for an additional 0.5 hours. To the suspension was added a cuprous bromide methyl sulphide complex (1.0 g, 0.0049 mol), and the reaction mixture was stirred for an additional 0.5 hours. A solution of 3-(4-methoxyphenyl) propyloxirane (4.12 g, 0.010 mol) in dry ether (10 ml) was added to the reaction mixture and the mixture was stirred for 1 hour at -78°C and then for an additional 12 hours at -25°C. The reaction mixture was allowed to warm up to room temperature overnight. To the reaction mixture was added aqueous ammonia solution (200 ml of 35%) and the resulting mixture was extracted with ethyl acetate (3 × 100 ml). The combined extracts were washed with brine (100 ml), dried over anhydrous sodium sulfate and concentrated under reduced pressure to yield a crude product. The crude product was purified by column chromatography (silica gel, 3% ethanol in dichloromethane) and recrystallized from ether-pentane to yield α-[3-(4-methoxyphenyl)propyl]-3-pyridineethanol as a colorless crystalline solid, having a melting point of 73-75°C and the following physical characteristics:

Elemental analysis: C,75.29%; H,7.81%; N,5.15%; as against calculated values of C,75.25%; H,7.80%; N,5.16% for $C_{17}H_{21}NO_2$.

$^1$H-NMR (δ-$CDCl_3$): 1.46-1.92 (m,4H), 2.06-2.34 (broad s,1H), 2.54-2.84 (m,2H), 2.60 (t,2H), 3.66-3.88 (m,1H), 3.78 (s,3H), 6.79 and 7.09 (ABq,4H), 7.15-7.24 (m,1H), 7.50-7.56 (m,1H) and 8.36-8.44 (m,2H); represented by the structural formula:

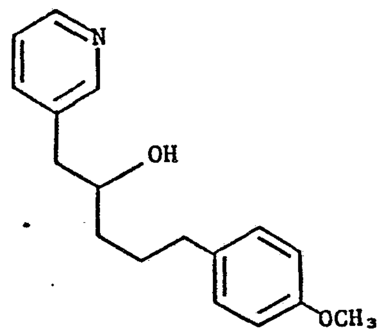

EXAMPLE 2

α-[3-(4-Methoxyphenyl)propyl]-3-pyridineethanol

a) 4-(4-Methoxyphenyl)butanal

A solution of dry dimethylsulphoxide (13.02g, 0.1667 mol) in dichloromethane (100 ml) was added dropwise over a period of 10 minutes to a stirred solution of oxalyl chloride (11.64g, 0.0917 mol) in dichloromethane (500 ml) at -50°C and the resulting mixture was stirred for 15 minutes. To the mixture was added a solution of 4-(4-methoxyphenyl)-1-butanol (15.0g, 0.0833 mol) in dichloromethane (50 ml) and the reaction mixture was stirred for an additional 15 minutes at -50°C. Triethylamine (42.07g, 0.4165 mol) was added to the reaction mixture and the resulting mixture was allowed to equilibrate to room temperature. The reaction mixture was diluted with dichloromethane (500 ml), washed with water (2 × 500 ml), then washed with a saturated aqueous solution of ammonium chloride (2 × 300 ml) and water (500 ml). The reaction mixture was dried over anhydrous sodium sulfate and concentrated under reduced pressure to yield 4-(4-methoxyphenyl)butanal as a pale yellow oil.

b) α-[3-(4-Methoxyphenyl)propyl]-3-pyridineethanol

n-Butyl lithium (28 ml of 1.6 M in hexane, 0.045 mol) was added to a stirred solution of diisopropylamine (4.55g, 0.045 mol) in dry tetrahydrofuran (50 ml) at 0°C under a nitrogen atmosphere and the resulting yellow solution was stirred at 0°C for 30 minutes. To the solution was added hexamethylphosphoramide (8.06g, 0.045 mol) and the reaction mixture was stirred for an additional 20 minutes at 0°C. 3-picoline (4.19g, 0.045 mol) was added to the reaction mixture and the resulting deep-red solution was stirred for 30 minutes at 0°C. To the reaction mixture was added 4-(4-methoxyphenol)butanal and the reaction mixture was stirred at 0° for an additional 30 minutes and then at room temperature for 60 minutes. The pale brown solution was extracted with hydrochloric acid (250 ml of 5M). The acid solution was made basic using potassium hydroxide and extracted with ethyl acetate (3 × 250 ml). The combined extracts were washed with water (3 × 250 ml), dried over anhydrous sodium sulfate, filtered, and concentrated under reduced pressure to yield a crude product. The crude product was purified by column chromatography (silica gel, chloroform) and recrystallized from ethyl acetate-pentane to yield α-[3-(4-methoxyphenyl)propyl] 3-pyridineethanol as a colorless crystalline solid, having a melting point of 73-75°C, and the following physical characteristics:

Elemental analysis: C,75.06%; H,7.79%; N,5.04%; as against calculated values of C,75.25%, H,7.80%; N,5.16% for $C_{17}H_{21}NO_2$.

$^1$H-NMR (δ-$CDCl_3$): 1.45-1.92 (m,4H), 2.06-2.34 (broad s,1H), 2.54-2.84 (m,2H), 2.60 (t,2H), 3.66-3.88 (m,1H), 3.78 (s,3H), 6.79 and 7.09 (ABq,4H), 7.15-7.24 (m,1H), 7.50-7.56 (m,1H) and 8.36-8.44 (m,2H); represented by the structural formula:

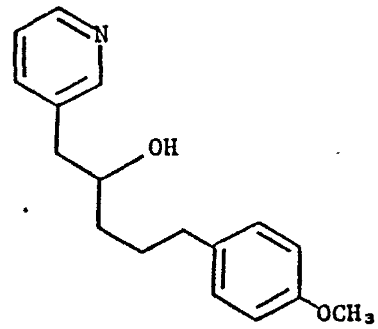

EXAMPLE 3

α-[3-(4-Methoxyphenyl)propyl]-3-pyridineethanol

a) 5-(4-Methoxyphenyl)-1-(3-pyridinyl)-2-pentanone

A solution of n-butyl lithium (28 ml of 1.6M in hexane, 0.124 mol) was added to a stirred solution of diisopropylamine (4.55 g, 0.045 mol) in dry tetrahydrofuran (50 ml) at 0°C under a nitrogen atmosphere and the resulting yellow solution was stirred at 0°C for 30 minutes. To the solution was added hexamethylphosphoramide (8.06g, 0.045 mol) and the resulting solution was stirred at 0°C for 15 minutes. 3-Picoline (4.19g, 0.045 mol) was added to the reaction mixture and the resulting deep-red solution was stirred at 0°C for 30 minutes. To the solution was added 4-(4-methoxyphenyl)butyric acid methyl ester (9.36g, 0.045 mol) in dry tetrahydrofuran (20 ml). The resulting mixture was stirred at 0°C for 15 minutes and then at room temperature for an additional 90 minutes. The pale-yellow solution was extracted with hydrochloric acid (300 ml of 1M). The acid solution was washed with ethyl acetate (2 × 250 ml), made basic using potassium hydroxide and extracted with ethyl acetate (4 × 200 ml). The combined extracts were dried over anhydrous sodium sulfate and the solvent was removed under reduced pressure to yield a crude product. The crude product was purified by column chromatography (silica gel, chloroform) and recrystallized from ethyl acetate-hexane to yield 5-(4-methoxyphenyl)-1-(3-pyridinyl)-2-pentanone as a colorless crystalline solid, having a melting point of 35-37°C and the following physical characteristics:

Elemental analysis: C,75.31%, H,7.03%, N,5.11%; as against calculated values of C,75.31%; %,7.14%, N,5.17% for $C_{17}H_{19}NO_2 \bullet 0.1 H_2O$.

$^1$H-NMR (δ-$CDCl_3$): 1.82-1.98 (m,2H), 2.46-2.62 (m,2H), 2.53 (t,2H), 3.67 (s,2H), 3.79 (s,3H), 6.83 and 7.06 (ABq,4H), 7.23-7.31 (m,1H), 7.29-7.37 (m,1H), 8.39-8.46 (m,1H) and 8.50-8.56 (m,1H).

b) α-[3-(4-Methoxyphenyl)propyl]-3-pyridineethanol

A stirred solution of 5-(4-methoxyphenyl)-1-(3-pyridinyl)-2-pentanone (2.0g, 0.0074 mol) in methanol (50 ml) at 0°C was treated with sodium borohydride (5.0g, 0.1: 2 mol) in portions over a period of 15 minutes. The mixture was stirred for an additional 15 minutes at 0°C and then allowed to equilibrate to room temperature. The solvent was removed under reduced pressure and the residue was extracted into ethyl acetate (250 ml). The ethyl acetate solution was washed with water (5 × 200 ml), dried over anhydrous sodium sulfate, filtered, and concentrated under reduced pressure to yield a crude product. The crude product was purified by column chromatography (silica gel, 20% hexane in chloroform) and recrystallized from ether-pentane to yield α-[3-(4-methoxyphenyl)propyl]-3-pyridineethanol as a colorless crystalline solid, having a melting point of 73-75°C and the following physical characteristics:

$^1$H-NMR (δ-$CDCl_3$): 1.46-1.92 (m,4H), 2.06-2.34 (broad s,1H), 2.54-2.84 (m,2H), 2.60 (t,2H), 3.66-3.88 (m,1H), 3.78 (s,3H), 6.79 and 7.09 (ABq,4H), 7.15-7.24 (m,1H), 7.50-7.56 (m,1H) and 8.36-8.44 (m,2H); and represented by the structural formula:

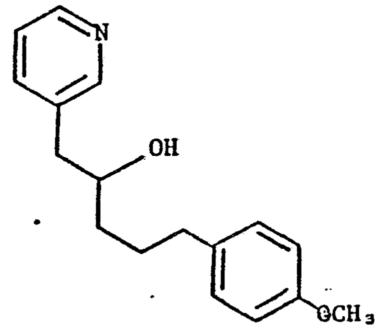

EXAMPLE 4

α-(3-Phenylpropyl)-3-pyridineethanol

a) 5-Phenyl-1-pentene

A solution of phenethyl magnesium bromide (prepared from the reaction of phenethyl bromide (25g., 0.135 mol) and excess magnesium turnings) in dry tetrahydrofuran (50 ml) was added to a stirred solution of allyl bromide (16.34g, 0.135 mol) in anhydrous tetrahydrofuran (150 ml) at room temperature and under a nitrogen atmosphere. The mixture was stirred at room temperature for three hours and the solvent was evaporated under reduced pressure and the resulting residue was dissolved in ethyl acetate (500ml). The solution was washed with a saturated aqueous solution of ammonium chloride (2 × 250 ml), dried over anhydrous magnesium sulfate and concentrated under reduced pressure to yield an oil which was distilled to yield 5-phenyl-1-pentene as a colorless oil (b.p. 40°C, 0.5 mm Hg) having the following physical characteristics:

$^1$H-NMR (δ-$CDCl_3$): 1.64-1.78 (m,2H), 2.00-2.14 (m,2H), 2.61 (t,2H), 4.89-5.07 (m,2H), 5.72-5.92 (m,1H), and 7.09-7.31 (m,5H).

b) 2-(3-Phenylpropyl)oxirane

A mixture of 5-phenyl-1-pentene (14.2g, 0.0973 mol) and 3-chloroperoxybenzoic acid (16.79g, 0.0973 mol) in dichloromethane (300 ml) were stirred at room temperature for four hours. The resulting solution was washed with a saturated aqueous sodium hydrogen carbonate solution (2 × 100 ml) and dried over anhydrous magnesium sulfate. The solvent was evaporated under reduced pressure to yield an oil which was distilled to yield 2-(3-phenylpropyl)oxirane (b.p. 85°C, 0.4 mm Hg) having the following physical characteristics:

$^1$H-NMR (δ-$CDCl_3$): 1.66-1.92 (m,4H), 2.45 (m,1H), 2.66 (t,2H), 2.73 (t,1H), 2.94 (m,1H) and 7.10-7.32 (m,5H).

c) α-(3-Phenylpropyl)-3-pyridineethanol

A solution of t-butyl lithium (7.2ml of 1.4M in hexane, 0.010 mol) was added with stirring to dry tetrahydrofuran (75 ml) at -100°C under a nitrogen atmosphere and stirred for 10 minutes. To the resulting yellow solution was added a solution of 3-bromopyridine (1.58g, 0.010 mol) in dry tetrahydrofuran (5 ml). The resulting green solution was stirred at -100°C for 20 minutes. To the solution was added a cuprous iodide trimethylphosphite complex (3.15 g, 0.010 mol) and the resulting pale brown solution was stirred at -78°C for 5 minutes. To the solution was added a solution of 2-(3-phenylpropyl)oxirane (1.62g, 0.010 mol) in dry tetrahydrofuran (5 ml) and the reaction mixture was then allowed to warm up to -20°C, stirred at -20°C for 60 minutes and then allowed to warm up to room temperature overnight. The solvent was evaporated under reduced pressure and the residue was dissolved in ethyl acetate (500 ml). The solution was washed with water (500 ml) and with saturated aqueous ammonium chloride solution (2 × 300 ml). The solution was

following physical characteristics:

$^1$H-NMR (δ-$CDCl_3$): 1.50-2.00 (m,4H), 2 00-2.10 (broad s, 1H), 2.60-2.86 (m, 2H), 2.66 (t,2H), 3.79-3.92 (m,1H), 7.14-7.35 (m,6H), 7.50-7.57 (m,1H) and 8.40-8.47 (m,2H); and represented by the structural formula:

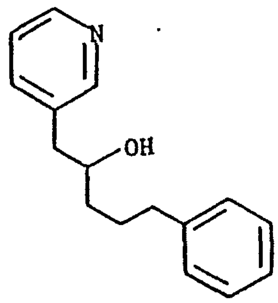

EXAMPLE 6

α-[3-(3,4-Dimethoxyphenyl)propyl]-3-pyridineethanol

a) Methanesulphonic acid, 2-(3,4-dimethoxyphenyl)ethyl ester

To a stirred solution of 2-(3,4-dimethoxyphenyl)ethanol (19.0g, 0.104 mol), triethylamine (20 ml, 0.198 mol) and 4-dimethylaminopyridine (0.050g, 0.0004 mol) in dichloromethane (100 ml) at -20°C was added dropwise methane sulfonyl chloride (12.0g, 0.105 mol) and the resulting mixture was stirred at 0°C for 3 hours. The reaction mixture was diluted with dichloromethane (300 ml), washed with a saturated aqueous solution of ammonium chloride (2 × 200 ml) and dried over anhydrous sodium sulfate. The solvent was evaporated under reduced pressure to yield methanesulphonic acid, 2-(3,4-dimethoxyphenyl)ethyl ester as an oil having the following characteristics:

$^1$H-NMR (δ-$CDCl_3$): 2.87 (s,3H), 2.99 (t,2H), 3.85 (s,3H), 3.87 (s,3H), 4.39 (t,2H) and 6.73-6.85 (m,3H).

b) 2-(3,4-Dimethoxyphenyl)ethyl bromide

A mixture of methanesulphonic acid, 2-(3,4-dimethoxyphenyl) ethyl ester (26.0g, 0.100 mol) and potassium bromide (30.0g, 0.250 mol) in dimethylformamide (400 ml) was stirred at 56°C for 11 hours. The solvent was evaporated off under reduced pressure at 50°C and the residue was extracted into dichloromethane (250 ml), washed with water (2 × 100 ml) and dried over anhydrous magnesium sulfate. The solvent was removed under reduced pressure to yield a crude product that was distilled (b.p. 90-95°C, 0.3 mm Hg) to yield 2-(3,4-dimethoxyphenyl)ethyl bromide as a colorless crystalline solid having the following physical characteristics:

$^1$H-NMR (δ-$CDCl_3$): 3.09 (t,2H), 3.54 (t,2H), 3.85 (s,3H), 3.87 (S,3H) and 6.70-6.80 (m,3H).

c) 5-(3,4-Dimethoxyphenyl)-1-pentene

2-(3,4-Dimethoxyphenyl)ethyl bromide (6.0g, 0.0245 mol) was added dropwise to a stirred mixture of magnesium powder (0.70g, 0.0288 mol) in dry tetrahydrofuran (60 ml) containing a single crystal of iodine, under a nitrogen atmosphere, at such a rate sufficient to maintain the mixture at reflux. When the addition of 2-(3,4-dimethoxyphenyl)ethyl bromide was complete, the resulting solution was stirred for 90 minutes at room temperature and then added to a solution of allyl bromide (4.5g, 0.0372 mol) in dry tetrahydrofuran (50 ml) at 0°C under a nitrogen atmosphere. The resulting mixture was allowed to warm up to room temperature overnight. The solvent was removed under reduced pressure and the residue was treated with

extracted with hydrochloric acid (3 × 200 ml of 1M). The acid solution was washed with ethyl acetate (200 ml), basified using sodium carbonate and extracted with ethyl acetate (3 × 200ml). The combined extracts were dried over anhydrous magnesium sulfate and the solvent was evaporated under reduced pressure to yield a crude product. The crude product was purified by column chromatography (silica gel, 5% ethanol in chloroform) and recrystallized from ether to yield α-(3-phenylpropyl)-3-pyridineethanol as a colorless crystalline solid, having a melting point of 62-64° and the following physical characteristics:

Elemental analysis: C,79.67%; H,7.93%; N,5.80%; as against calculated values of C,79.63%; H,7.94%; N,5.80% for $C_{16}H_{19}NO$.

$^1$H-NMR (δ-$CDCl_3$): 1.50-2.00 (m,4H), 2.00-2.10 (broad s, 1H), 2.60-2.86 (m,2H), 2.66 (t,2H), 3.79-3.92 (m,1H), 7.14-7.35 (m,6H), 7.50-7.57 (m,1H) and 8.40-8.47 (m,2H); and represented by the structural formula:

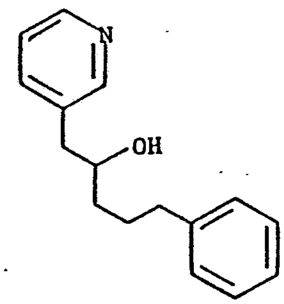

EXAMPLE 5

α-(3-Phenylpropyl)-3-pyridineethanol

a) 5-phenyl-1-(3-pyridinyl)-2-pentanone

n-Butyl lithium (3.9 ml of 1.6M in hexane, 0.00624 mol) was added to a stirred solution of diisopropylamine (0.60g, 0.00590 mol) in dry tetrahydrofuran (30 ml) at 0°C under a nitrogen atmosphere. The resulting yellow solution was stirred at 0°C for a 15 minutes, and the cooled to -78°C. To the solution was dropwise added a solution of 3-pyridinyl-2-propanone (0.40g, 0.00296 mol) in dry tetrahydrofuran (5 ml) over a period of 10 minutes. The resulting orange solution was stirred at -78°C for 1 hour and a solution of phenethylbromide (0.55g, 0.00296 mol) in dry ether (3 ml) was then added. The mixture was stirred at -78°C for 1 hour and then allowed to warm to room temperature over a period of 5 hours. The reaction was quenched and extracted with hydrochloric acid (50 ml of 2M). The acid solution was washed with ether (2 × 50 ml), made basic (pH 8) with sodium hydroxide and extracted with dichloromethane (3 × 100 ml). The combined extracts were dried over anhydrous magnesium sulfate and the solvent was evaporated under reduced pressure to yield a crude product. The crude product was purified by column chromatography (silica gel, 6% methanol in ethyl acetate) to yield 5-phenyl-1-(3-pyridinyl)-2-pentanone as a colorless oil having the following structural characteristics:

$^1$H-NMR (δ-$CDCl_3$): 1.88 (dt, 2H), 256 (m,4H), 3.72 (s,2H), 6.9 (m,5H), 7.26 (m,1H), 7.52 (dd,1H), 8.48 (dd,1H) and 8.58 (m,1H).

b) α-(3-Phenylpropyl)-3-pyridineethanol

Utilizing the procedure described in Example 3(b) but employing 5-phenyl-1-(3-pyridinyl)-2-pentanone in lieu of 5-(4-methoxyphenyl)-1-(3-pyridinyl)-2-pentanone, yielded a crude product which was purified by column chromatography (silica gel, 5% ethanol in chloroform) and recrystallized from ether to yield α-(3-phenylpropyl)-3-pyridineethanol as a colorless crystalline solid, having a melting point of 62-64°C and the

a saturated aqueous solution of ammonium chloride (150 ml) and extracted with dichloromethane (3 × 150 ml). The combined dichloromethane extracts were dried over anhydrous sodium sulfate, filtered, and concentrated under reduced pressure to yield a crude product. The crude product was purified by column chromatography (silica gel, dichloromethane) to yield 5-(3,4-dimethoxyphenyl)-1-pentene as a colorless oil (b.p. 178-182°C, 0.5 mm Hg) having the following physical characteristics:

$^1$H-NMR (δ-$CDCl_3$): 1.54-1.67 (m,2H), 2.04-2.16 (m,2H), 2.57 (t,2H), 3.84 (s,3H), 3.87 (s,3H), 4.94-5.06 (m,2H), 5.73-5.92 (m,1H), 6.69-6.81 (m,3H).

d) 3-(3,4-Dimethoxyphenyl)propyloxirane

To a stirred solution of 5-(3,4-dimethoxyphenyl)-1-pentene (5.30g, 0.0257 mol) in dichloromethane (50 ml) at 0°C was added 3-chloroperoxybenzoic acid (6.50g of 80%, 0.0301 mol) and the resulting mixture was allowed to warm up to room temperature overnight. Dichloromethane (200 ml) was added to the reaction mixture and the resulting solution was washed with saturated aqueous sodium hydrogen carbonate solution (3 × 200 ml), dried over anhydrous sodium sulfate, filtered, and concentrated under reduced pressure to yield a crude product. The crude product was purified by column chromatography (silica gel, dichloromethane) to yield 3-(3,4-dimethoxyphenyl)propyloxirane as a colorless oil having the following physical characteristics:

$^1$H-NMR (δ-$CDCl_3$): 1.46-1.86 (m,4H), 2.45-2.49 (m,1H), 2.62 (t,2H), 2.75 (t,1H), 2.89-3.00 (m,1H), 3.86 (s,3H), 3.87 (s,3H) and 6.70-6.82 (m,3H).

e) α-[3-(3,4-Dimethoxyphenyl)propyl]-3-pyridineethanol

Utilizing the procedure described in Example 1 (c) but employing 3-(3,4-dimethoxyphenyl)propyloxirane in lieu of 3-(4-methoxyphenyl)propyloxirane, yielded a crude product which was purified by column chromatography (silica gel, 2% ethanol in chloroform) and recrystallized from ether to yield α-[3-(3,4-dimethoxyphenyl)propyl]-3-pyridineethanol as colorless crystalline needles, having a melting point of 74-76°C and the following physical characteristics:

Elemental analysis: C,72.05%; H,8.17%; N,4.65%, as against calculated values of C,71.73%; H,7.69%; N,4.65% for $C_{18}H_{23}NO_3$.

$^1$H-NMR (δ-$CDCl_3$): 1.49-1.93 (m,4H), 2.60 (t,2H), 2.50-2.80 (m,2H), 3.0 (broad s,1H), 3.75-3.90 (m,1H), 3.86 (s,3H), 3.87 (s,3H), 6.65-6.82 (m,3H), 7.14-7.22 (m,1H), 7.48-7.56 (m,1H) and 8.30-8.38 (m,2H); and represented by the structural formula:

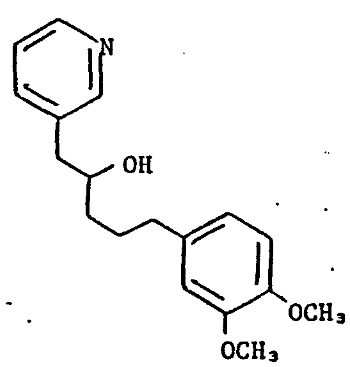

EXAMPLE 7

α-[2-(4-Methoxyphenyl)ethyl]-3-pyridineethanol

a) 4-(4-Methoxyphenyl)-1-butene

To a stirred solution of 4-methoxybenzyl bromide (8.55g, 0.044 mol) and a cuprous bromide methyl sulphide complex (0.20g, 0.0010 mol) in dry tetrahydrofuran (80 ml) at -40°C under a nitrogen atmosphere, was added a solution of allyl magnesium bromide (100 ml of 1.0M in tetrahydrofuran, 0.100 mol). The resulting solution was stirred at -20°C for 12 hours. The solution was allowed to warm to room temperature, treated with a saturated aqueous solution of ammonium chloride (200 ml) and extracted with ether (3 × 150 ml). The combined extracts were dried over anhydrous magnesium sulfate, filtered, and concentrated under reduced pressure to yield an oil which was purified by distillation under reduced pressure to yield 4-(4-methoxyphenyl)-1-butene as a colorless oil having the following physical characteristics:

$^1$H-NMR (δ-$CDCl_3$): 2.25-2.40 (m,2H), 2.65 (t,2H), 3.78 (s,3H), 4.92-5.08 (m,2H), 5.70-5.94 (m,1H), 6.85 and 7.09 (ABq, 4H).

b) 2-(4-Methoxyphenyl)ethyloxirane

Utilizing the procedure described in Example 1 (b) but employing 4-(4-methoxyphenyl)-1-butene *in lieu* of 5-(4-methoxyphenyl)-1-pentene, yielded a crude product which was purified by column chromatography (silica gel, dichloromethane) to yield 2-(4-methoxyphenyl)ethyloxirane as a colorless oil having the following physical characteristics:

$^1$H-NMR (δ-$CDCl_3$): 1.76-1.88 (m,2H), 2.43-2.48 (m,1H), 2.63-2.86 (m,1H), 2.72 (t,2H), 2.89-3.00 (m,1H), 3.79 (s,3H), 6.83 and 7.13 (ABq,4H).

c) α-[2-(4-Methoxyphenyl)ethyl]-3-pyridineethanol

Utilizing the procedure described in Example 1 (c) but employing 2-(4-methoxyphenyl)ethyloxirane *in lieu* of 3-(4-methoxyphenyl)propyloxirane, yielded a crude product which was purified by column chromatography (silica gel, dichloromethane) to yield α-[2-(4-methoxyphenyl)ethyl]-3-pyridineethanol as an oil, which solidified on standing (m.p. 56°C), having the following physical characteristics:

Elemental analysis: C,73.55%, H,7.28%; N,5.50%; as against calculated values of C,73.39%; H,7.51%; N,5.35% for $C_{16}H_{19}NO_2 \bullet 0.25H_2O$.

$^1$H-NMR (δ-$CDCl_3$): 1.75-1.85 (m,2H), 2.39 (broad s,1H), 2.64-2.86 (m,4H), 3.76-3.85 (m,1H), 3.78 (s,3H), 6.83 and 7.11 (ABq,4H), 7.17-7.23 (m,1H), 7.51-7.56 (m,1H), and 8.37-8.43 (m,2H); and represented by the structural formula:

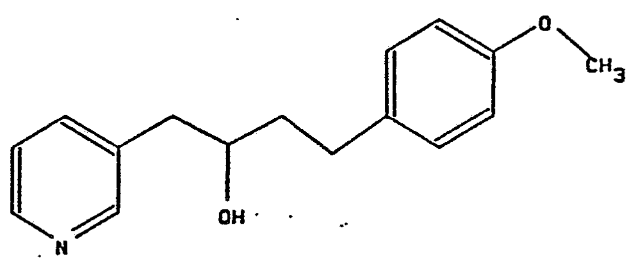

+ 0.25 H2O

## EXAMPLE 8

α-[4-(4-Methoxyphenyl)butyl]-3-pyridineethanol

a) 6-(4-Methoxyphenyl)-1-hexene

5-Bromopentene (5.0g, 0.0335 mol) was added dropwise to magnesium turnings (1.0g, 0.0429 mol) in dry tetrahydrofuran (50 ml) containing a single crystal of iodine, under a nitrogen atmosphere, at such rate sufficient to maintain the mixture at reflux. when the addition of 5-bromopentene was complete, the resulting mixture was heated at 60°C for 1 hour and then cooled to 0°C. To the solution was added 4-methoxybenzyl chloride (8.5g, 0.0543 mol) and the reaction mixture was allowed to warm to room temperature overnight. The resulting mixture was extracted with a saturated aqueous ether (3 × 150 ml). The combined extracts were dried over anhydrous sodium carbonate, filtered, and concentrated under reduced pressure to yield a crude product. The crude product was purified by column chromatography (silica gel, 10% ether in hexane) to yield 6-(4-methoxyphenyl)-1-hexene as a colorless oil having the following physical characteristics:

$^1$H-NMR (δ-$CDCl_3$): 1.23-1.50 (m,2H), 1.50-1.68 (m,2H), 2.00-2.12 (m,2H), 2.54 (t,2H), 3.75 (s,3H), 4.87-5.04 (m,2H), 5.69-5.90 (m,1H), 6.79 and 7.06 (ABq,4H).

b) 4-(4-Methoxyphenyl)butyloxirane

Utilizing the procedure described in Example 6 (d) but employing 6-(4-methoxyphenyl)-1-hexene in lieu of 5-(3,4-dimethoxyphenyl)-1-pentene, yielded a crude product which was purified by column chromatography (silica gel, 10% ether in hexane) to yield 4-(4-methoxyphenyl)butyloxirane as a pale yellow oil having the following physical characteristics:

$^1$H-NMR (δ-$CDCl_3$): 1.52-1.72 (m,6H), 2.42-2.48 (m,1H), 2.58 (t,3H), 2.74 (t,1H), 2.84-2.96 (m,1H), 3.76 (s,3H), 6.81 and 7.08 (ABq,4H).

c) α-[4-(4-Methoxyphenyl)butyl]-3-pyridineethanol

Utilizing the procedure described in Example 1 (c) but employing 4-(4-methoxyphenyl)butyloxirane in lieu of 3-(4-methoxyphenyl)propyloxirane, yielded a crude product which was purified by column chromatography (silica gel, chloroform) and recrystallized from ether-pentane to yield α-[4-(4-methoxyphenyl)butyl]-3-pyridineethanol as a colorless crystalline solid, having a melting point of 60-62°C and the following physical characteristics:

Elemental analysis: C,75.52%; H,7.96%; N,4.85%; as against calculated values of C,75.76%; H,8.12%; N,4.91% for $C_{18}H_{23}NO_2$.

$^1$H-NMR (δ-$CDCl_3$): 1.42-1.73 (m,6H), 2.28 (broad s,1H), 2.58 (t,2H), 2.59-2.85 (m,2H), 3.73-3.88 (m,1H), 3.78 (s,3H), 6.81 and 7.09 (ABq,4H), 7.15-7.23 (m,1H) 7.49-7.58 (m,1H) and 8.37-8.44 (m,2H); and represented by the structural formula:

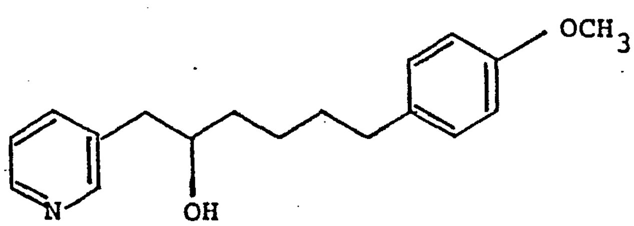

EXAMPLE 9

α-[3-(4-Methoxyphenyl)propyl]-4-pyridineethanol

Utilizing the procedure described in Example 1 (c) but employing 4-bromopyridine in lieu of 3-bromopyridine, yielded a crude product which was purified by column chromatography (silica gel, 5% ethanol in chloroform) and recrystallized from benzene-pentane to yield α-[3-(4-methoxyphenyl)propyl]-4-pyridineethanol as a colorless crystalline solid, having a melting point of 72.5-74°C and the following physical characteristics:

Elemental analysis: C,75.39%, H,8.19%; N, 5.22%; as against calculated values of C,75.25%; H,7.80%; N,5.16% for $C_{17}H_{21}NO_2$.

$^1$H-NMR (δ-$CDCl_3$): 1.48-1.92 (m,4H), 2.56-2.84 (m,2H), 2.58 (t,2H), 2.89 (broad s,1H), 3.78 (s,3H), 3.78-3.92 (m,1H), 6.82 and 7.12 (ABq,4H) and 7.08 and 8.37 (ABq,4H); and represented by the structural formula:

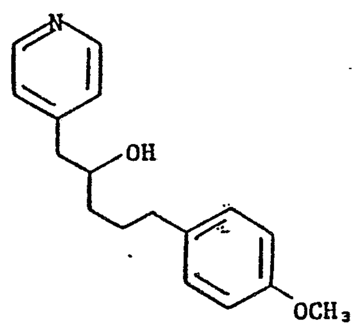

EXAMPLE 10

α-Methyl-α-[3-(4-Methoxyphenyl)propyl]-3-pyridineethanol

a) 3-(4-Methoxyphenyl)propyl bromide

A solution of thionyl bromide (22g, 0.105 mol) in dichloromethane (50 ml) was added dropwise to a stirred solution of 4-methoxyphenylpropanol (16.6g, 0.100 mol) in dichloromethane (100 ml). To the solution was added dimethylformamide (1.2 ml) and the resulting solution was heated under reflux conditions for 4.5 hours. The solution was diluted with dichloromethane (300 ml), washed with a saturated aqueous solution of sodium metabisulphite (2×150 ml) and saturated aqueous sodium hydrogen carbonate solution (2×150 ml) and dried over sodium sulfate. The solvent was removed under reduced pressure to yield 3-(4-methoxyphenyl)propyl bromide as a pale yellow oil, having the following physical characteristics:

$^1$H-NMR (δ-$CDCl_3$): 2.13 (h,2H), 2.73 (t,2H), 3.38 (t,2H), 3.78 (s,3H), 683 and 7.11 (ABq,4H).

b) 5-(4-methoxyphenyl)-2-pentanol

A solution of 3-(4-methoxyphenyl)propyl bromide (6.8g, 0.030 mol) in dry tetrahydrofuran (5 ml) was added dropwise to magnesium turnings (1.08g, 0.044 mol) in dry tetrahydrofuran (40 ml) containing a single crystal of iodine, under a nitrogen atmosphere, at a rate sufficient to maintain the mixture at reflux. When the addition of 3-(4-methoxyphenyl)propyl bromide was complete, the mixture was heated at 60°C for 1 hour. The solution was cooled to -10°C and a solution of acetaldehyde (3.52g, 0.080 mol) in dry tetrahydrofuran was added. The resulting solution was allowed to warm up to room temperature and was stirred for 1 hour, after which was added a saturated aqueous solution of ammonium chloride (200 ml). The resulting mixture was extracted with ether (3×150 ml). The combined ether extracts were washed with water (100 ml), dried over anhydrous sodium sulfate, filtered, and concentrated under reduced pressure to yield a crude product. The crude product was purified by column chromatography (silica gel, 10% ethanol in dichloromethane) to yield 5-(4-methoxyphenyl)-2-pentanol as a colorless oil.

c) 5-(4-Methoxyphenyl)-2-pentanone

Pyridinium chlorochromate (10.3g, 0.0048 mol) was added to dichloromethane (200 ml) and the resulting solution was stirred vigorously for 15 minutes. A solution of 5-(4-methoxyphenyl)-2-pentanol (6.2g, 0.032 mol) in dichloromethane (50 ml) was added and the resulting mixture was stirred for 1.5 hours at room temperature. The solution was filtered through a filtering aid, washing with dichloromethane (100 ml). The filtrate and washings were combined and the solvent was removed under reduced pressure to give a residue that was dissolved in ether (200 ml), washed with hydrochloric acid (2×100ml of 2M) followed by water (100 ml) and dried over anhydrous sodium sulfate. The solvent was removed under reduced pressure to yield 5-(4-methoxyphenyl)-2-pentanone as a pale yellow oil, having the following physical characteristics:

$^1$H-NMR (δ-$CDCl_3$): 1.85-1.94 (h,2H), 2.11 (s,3H), 2.42 (t,2H), 2.55 (t,2H), 3.78 (s,3H), 6.68 and 7.08 (ABq, 4H).

d) α-Methyl-α-[4-(3-methoxyphenyl)propyl]-3-pyridineethanol

Utilizing the procedure described in Example 2 (b) but employing 5-(4-methoxyphenyl)-2-pentanone in lieu of 4-(4-methoxyphenyl)butanal, yielded a crude product which was purified by column chromatography (silica gel, chloroform) and distillation (b.p. 150°C, 0.03 mm. Hg) to yield α-methyl-α-[4-(3-methoxyphenyl)-propyl]-3-pyridineethanol as a colorless oil, having the following physical characteristics:

Elemental analysis: C,75.52%; H,8.14%;, N,4.83% as against calculated values of C,75.75%;, H,8.12%; N,4.91% for $C_{18}H_{23}NO_2$.

$^1$H-NMR (δ-$CDCl_3$): 1.12 (s,3H), 1.45-1.80 (m,4H), 2.55 (t,2H), 2.68 and 2.75 (ABq,2H), 3.47 (s,1H), 3.78 (s,3H), 6.81 and 7.08 (ABq,4H), 7.15-7.20 (m,1H), 7.45-7.55 (m,1H) and 8.42-8.50 (m,2H) and represented by the structural formula:

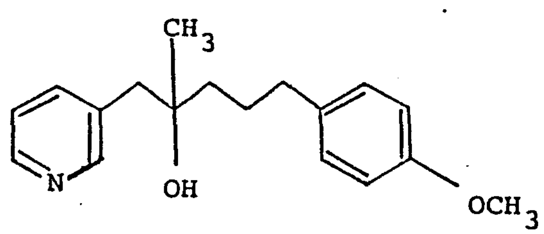

EXAMPLE 11

α-[3-(4-Methoxyphenyl)ethyl]-3-pyridinepropanol

a) 3-(3-Pyridinyl)propanal

Utilizing the procedure described in Example 2 (a) but employing 3-(3-pyridinyl)-1-propanol in lieu of 4-(4-methoxyphenyl)-1-butanol, yielded 3-(3-pyridinyl)propanal as a pale yellow oil, having the following physical characteristics:

$^1$H-NMR (δ-$CDCl_3$): 2.81 (t,2H), 2.95 (t,2H), 7.18-7.25 (dd,1H), 7.48-7.55 (m,1H), 8.38-8.50 (m,2H) and 9.80 (s,1H).

b) α--[3-(4-Methoxyphenyl)ethyl]-3-pyridinepropanol

A solution of 2-(4-methoxyphenyl)ethyl bromide (9.55g, 0.0444 mol) in dry tetrahydrofuran (10 ml) was added dropwise to magnesium turnings (1.08g, 0.0444 mol) in dry tetrahydrofuran (20 ml) containing a single crystal of iodine, under a nitrogen atmosphere, at such a rate sufficient to maintain the reaction mixture at reflux. When the addition was complete, the resulting solution was heated at 60°C for an additional 1.5 hours. The solution was cooled to 0°C and a solution of 3-(3-pyridinyl)propanal (2.0g, 0.0148 mol) in dry tetrahydrofuran (10 ml) was added. The resulting solution was allowed to warm to room temperature and stirred for an additional 1 hour, after which was added a saturated aqueous solution of ammonium chloride (50 ml). The resulting mixture was extracted with ethyl acetate (2×250 ml). The combined extracts were washed with water (2×250 ml) and extracted with hydrochloric acid (3×200 ml of 1M). The acid solution was washed with ethyl acetate (100 ml), made basic using sodium carbonate and extracted with ethyl acetate (4×200 ml). The combined extracts were dried over sodium sulfate, filtered and concentrated under reduced pressure to yield a crude product. The crude product was purified by column chromatography (silica gel, chloroform) to yield α-[3-(4-methoxyphenyl)ethyl]-3-pyridinepropanol as a pale yellow oil, having the following physical characteristics:

Elemental analysis: C,74.94%; H,7.85%; N,5.01; as against calculated values of C,75.25%; H,7.80%; N,5.16% for $C_{17}H_{21}NO_2$.

$^1$H-NMR (δ-$CDCl_3$): 1.73-1.85 (m,4H), 2.55-2.88 (m,5H), 3.58-3.70 (m,1H), 3.78 (s,3H), 6.82 and 7.08 (ABq,4H), 7.14-7.21 (m,1H), 7.45-7.53 (m,1H) and 8.38-8.45 (m,2H); and represented by the structural formula:

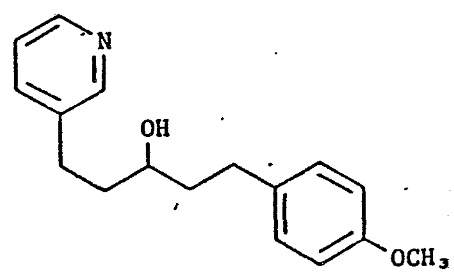

EXAMPLE 12

α-[3-(4-Methoxyphenyl)propyl]-3-pyridinepropanol

Utilizing the procedure described in Example 20(b) but employing 3-(4-methoxyphenyl)propyl bromide *in lieu* of 2-(4-methoxyphenyl)ethyl bromide yielded a crude product which was purified by column chromatography (silica gel, 2% methanol in ethyl acetate) to yield α-[3-(4-methoxyphenyl)propyl]-3-pyridinepropanol as a colorless oil, having the following physical characteristics:

Elemental analysis: C,75.98%; H,8.12%; N,5.32% as against calculated values of C,75.76%; H,8.12%; N,4.91% for $C_{18}H_{23}NO_2$.

$^1$H-NMR (δ-$CDCl_3$): 1.46-1.86 (m,6H), 2.07 (broad s,1H), 2.57 (t,2H), 2.60-2.88 (m,2H), 3.58-3.69 (m,1H), 3.78 (s,3H) 6.82 and 7.08 (ABq,4H), 7.16-7.24 (m,1H), 7.46-7.55 (m,1H) and 8.39-8.47 (m,2H); and represented by the structural formula:

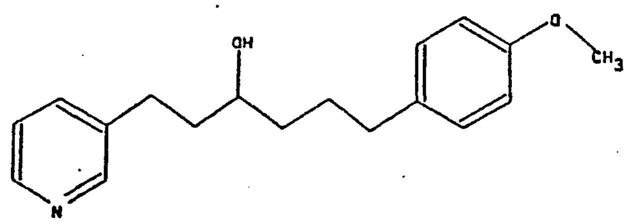

EXAMPLE 13

α-[3-(4-Methylthiophenyl)propyl]-3-pyridineethanol

a) 4-(4-Methylthiophenyl)-3-oxobutanoic acid

Aluminum chloride (66.7g, 0.50 mol) was added to a stirred mixture of freshly distilled thioanisole (37.3g, 0.30 mol) and succinic anhydride (30.0g, 0.30 mol) in nitromethane (200 ml) at 0°C. Stirring was maintained for 10 hours and the mixture was poured onto iced water (1000 ml). The resulting mixture was filtered and the filtrate was evaporated to dryness under reduced pressure. The residue was dissolved in hot saturated aqueous sodium carbonate solution, filtered and acidified with hydrochloric acid. The precipitated crude product was filtered off, dried and recrystallised from toluene to yield 4-(4-methylthiophenyl)-3-oxobutanoic acid as a colorless crystalline solid, having a melting point of 155-157°C.

b) 4-(4-Methylthiophenyl)butanoic acid

A solution of 4-(4-methylthiophenyl)-3-oxobutanoic acid (20g, 0.089 mol), hydrazine hydrate (20g of 80% aqueous solution, 0.32 mol) and potassium hydroxide (18g, 0.32 mole in diethylene glycol (80 ml) was heated under reflux for 6 hours with azeotropic removal of water. The solution was concentrated under reduced pressure and the residue was dissolved in water (250 ml) and acidified with hydrochloric acid. The precipitated crude product was collected by filtration, dried and recrystallized from water to yield 4-(4-methylthiophenyl)butanoic acid as a colorless crystalline solid, having a melting point of 54°C and the following physical characteristics:

$^1$H-NMR(δ-$CDCl^3$): 1.92 (p,2H), 2.35 (t,2H), 2.48 (s,3H), 2.63 (t,2H), 7.11 and 7.20 (ABq,4H) and 8.06 (broad s,1H).

c) 4-(4-Methylthiophenyl)butyryl chloride

Oxalyl chloride (20g, 0.158 mol) was added to a stirred solution of 4-(4-methylthiophenyl)butanoic acid (109, 0.0476 mol) and dimethylformide (0.2 ml) in benzene (100 ml). The resulting solution was stirred at room temperature for 3 hours. The solvent and excess reagent were removed under reduced pressure to yield crude 4-(4-methylthiophenyl) butyryl chloride as a colourless oil that was used without further purification.

d) 5-(4-Methylthiophenyl)-1-(3-pyridinyl)-2-pentanone

n-Butyl lithium (33.4 ml of 2.5M in hexane, 0.0835 mol) was added to a stirred solution of diisopropylamine (8.43g, 0.0835 mol) in dry tetrahydrofuran (250 ml) at 0°C under a nitrogen atmosphere. The resulting yellow solution was stirred at 0°C for a further 30 minutes, treated with 3-pyridylacetic acid (5.20g, 0.038 mol) and stirred at 45°C for 1 hour. The resulting yellow solution was then cooled to -78°C, treated with 4-(4-methylthiophenyl)butyryl chloride (4.50g, 0.020 mol) and stirred for 1 hour at -78°C. The mixture was then allowed to warm up to room temperature over 3 hours and quenched with hydrochloric acid (400 ml of 4M). The solution was washed with ether (2×100ml), then adjusted to pH 9 with aqueous ammonia solution and extracted with dichloromethane (4×100ml). The combined extracts were dried over

anhydrous magnesium sulphate and the solvent was removed under reduced pressure to yield a crude product. The crude product was purified by column chromatography (silica gel, chloroform) to yield 5-(4-methylthiophenyl)-1-(3-pyridinyl)-2-pentanone as a pale yellow oil, having the following physical characteristics:

$^1$H-NMR($\delta$-$CDCl_3$): 1.89 (p,2H), 2.47 (s,3H), 2.46-2.59 (m,4H), 3.66 (s,2H), 7.06 and 7.19 (ABq,4H), 7.26 (dd,1H), 7.48-7.54 (m,1H), 8.42 (d,1H) and 8.52 (dd,1H).

e) α-[3-(4-Methylthiophenyl)propyl]-3-pyridineethanol

Utilising the procedure described in Example 3(b) but employing 5-(4-methylthiophenyl)-1-(3-pyridinyl)-2-pentanone in lieu of 5-(4-methoxyphenyl)-1-(3-pyridinyl)-2-pentanone, yielded a crude product that was purified by column chromatography (silica gel, 5% methanol in ethyl acetate) and recrystallized from acetone-hexane to yield α-[3-(4-methylthiophenyl)propyl]-3-pyridineethanol as a colorless solid, having a melting point of, 69-70°C and the following physical characteristics:

Elemental analysis: C,71.48%; H,7.64%; N,4.74%; as against calculated values of C,71.04%; H,7.36%; N,4.87% for $C_{17}H_{21}NOS$.

$^1$H-NMR ($\delta$-$CDCl_3$): 1.46-1.90 (m,4H), 2.10 (broad s, 1H), 2.47 (s,3H), 2.60-2 85 (m,2H), 2.60 (t,2H), 3.76-3.88 (m,1H), 7.10 and 7.22 (ABq,4H), 7.15-7.24 (m,1H), 7.50-7.56 (m,1H) and 8.38-8.47 (m,2H); and represented by the structural formula:

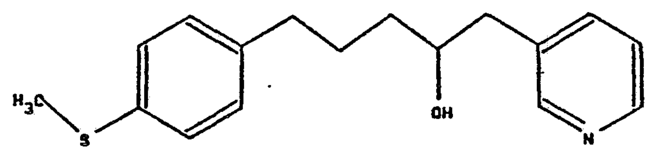

EXAMPLE 14

PAF - Induced Primary Aggregation in Human Platelet-Rich Plasma

Human venous blood was collected from healthy male donors, who had denied any medication during the previous 14 days. Nine volumes of blood were mixed with one volume of 3.24% trisodium citrate. The citrated blood was centrifuged at 160 g for ten minutes at 22°C to obtain platelet rich plasma (PRP). The platelets were then counted on a Coulter counter, and the platelet count was adjusted to 200,000 per µl with plasma. The PRP was then treated with indomethacin dissolved in dimethylsufoxide (10 µg indomethacin per ml PRP). A stock solution of PAF ($C_{16}$ or $C_{18}$ β-acetyl-γ-O-alkyl-L-α-phosphatidylcholine; 1 mg/ml) was prepared as a solution in chloroform-methanol (9:1 v/v). A 10 µl portion of the stock solution was evaporated to dryness under nitrogen and dissolved in 0.15M sodium chloride-0.15M Tris•HCl (90:10 v/v) buffer containing 0.25% bovine albumin. Small volumes of the PAF solution (5-15 µl) were added to 0.5 ml samples of indomethacin-treated PRP and the aggregation trace was recorded using an aggregometer. A dose response curve was obtained and a sub-optimal dose of PAF was determined for use in inhibition studies.

Indomethacin-treated PRP (0.5 ml) was incubated with a test compound dissolved in buffer (ca. pH) or dimethylsulfoxide for two minutes at 37°C prior to addition of the sub-optimal dose of PAF. The subsequent aggregation trace was recorded and the percent inhibition of PAF primary aggregation was determined by comparison with a control PAF-induced aggregation trace. (Due to variability in PAF-induced aggregation within a given PRP sample, a control PAF-induced aggregation was determined every two or three samples.) An inhibition dose-response curve was determined for each test compound and the $IC_{50}$ value calculated. Table I lists $IC_{50}$'s for illustrative examples of the compounds of this invention.

Table I. PAF-induced primary aggregation in human platelet-rich plasma

| Compound [Example number] | $IC_{50}$ (µM) |
|---|---|
| 1 | 53 |
| 4 | 83 |
| 6 | 36 |
| 7 | 71 |
| 8 | 208 |
| 9 | 525 |
| 10 | 166 |
| 11 | 60 |
| 12 | 231 |

## Claims

1. A compound of the formula

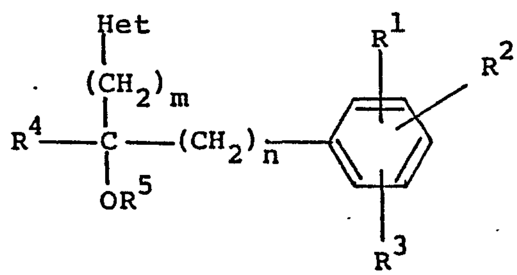

wherein $R^1$, $R^2$ and $R^3$ are independently hydrogen, $C_1$-$C_8$ alkoxy, or $C_1$-$C_8$ alkylthio;

$R^4$ is hydrogen or $C_1$-$C_8$ alkyl;

$R^5$ is hydrogen, $-\overset{O}{\overset{\|}{C}}-R^6$ or $C_1$-$C_8$ alkyl;

wherein $R^6$ is $C_1$-$C_8$ alkyl;

n and m are independently integers of from 1 to 5;

and Het is pyridyl; or a pharmaceutically acceptable acid addition salt thereof.

2. A compound according to Claim 1 wherein $R^5$ is hydrogen.

3. A compound according to Claim 2 having the formula

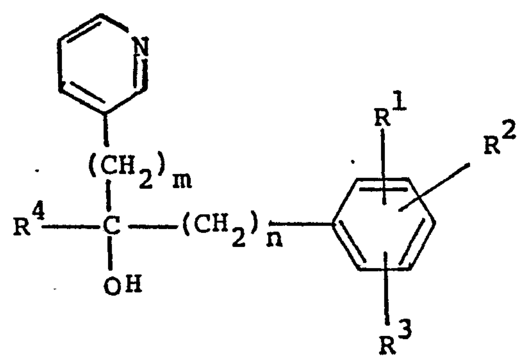

or a pharmaceutically acceptable acid addition salt thereof.

4. A compound according to Claim 3 wherein $R^4$ is hydrogen.

5. A compound according to Claim 4 having the formula

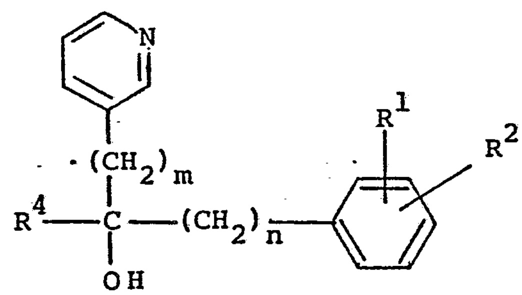

wherein one of $R^1$ and $R^2$ is $C_1$-$C_8$ alkoxy and the other of $R^1$ and $R^2$ is hydrogen or $C_1$-$C_8$ alkoxy; or a pharmaceutically acceptable acid addition salt thereof.

6. A compound according to Claim 5 wherein m is 1.

7. A compound according to Claim 6 wherein n is 2, 3, or 4.

8. A compound according to Claim 7 which is α-[3-(4-methoxyphenyl)propyl]-3-pyridineethanol.

9. A compound according to Claim 7 which is α-[2-(4-methoxyphenyl)ethyl]-3-pyridineethanol.

10. A compound according to Claim 7 which is α-[4-(4-methoxyphenyl)butyl]-3-pyridineethanol.

11. A compound according to Claim 7 which is α-[3-(3,4-dimethoxyphenyl)propyl]-3-pyridineethanol.

12. A compound according to Claim 5 wherein m is 2.

13. A compound according to Claim 12 wherein n is 2 or 3.

14. A compound according to Claim 13 which is α-[3-(4-methoxyphenyl)ethyl]-3-pyridinepropanol.

15. A compound according to Claim 13 which is α-[3-(4-methoxyphenyl)propyl]-3-pyridinepropanol.

16. A compound according to Claim 4 having the formula

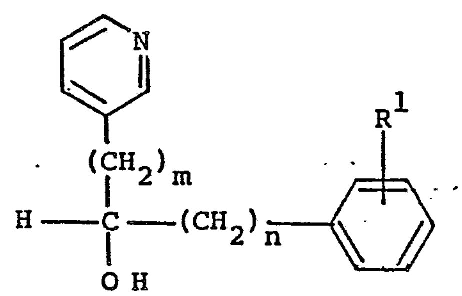

wherein $R^1$ is $C_1$-$C_8$ alkylthio; or a pharmaceutically acceptable acid addition salt thereof.

17. A compound according to Claim 16 wherein m is 1.

18. A compound according to Claim 16 wherein n is 3.

19. A compound according to Claim 16 which is α-[3-(4-methylthiophenyl)propyl]-3-pyridineethanol.

20. A compound according to Claim 4 having the formula

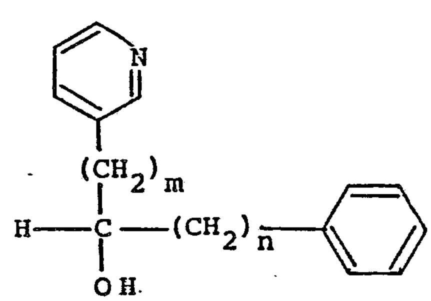

or a pharmaceutically acceptable acid addition salt thereof.

21. A compound according to Claim 20 wherein m is 1.

22. A compound according to Claim 20 wherein n is 3.

23. A compound according to Claim 20 which is α-(3-phenylpropyl)-3-pyridineethanol.

24. A compound according to Claim 3 wherein $R^4$ is $C_1$-$C_8$ alkyl.

25. A compound according to Claim 24 wherein one of $R^1$, $R^2$ and $R^3$ is $C_1$-$C_8$ alkoxy and the others of $R^1$, $R^2$ and $R^3$ are hydrogen.

26. A compound according to Claim 24 wherein m is 1.

27. A compound according to Claim 24 wherein n is 3.

28. A compound according to Claim 24 which is α-methyl-α-[3-(4-methoxyphenyl)propyl]-3-pyridineethanol.

29. A compound according to Claim 2 having the formula

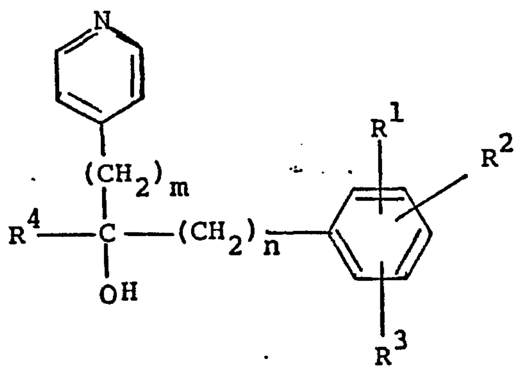

or a pharmaceutically acceptable acid addition salt thereof.

30. A compound according to Claim 29 wherein $R^4$ is hydrogen.

31. A compound according to Claim 29 wherein one of $R^1$, $R^2$ and $R^3$ is $C_1$-$C_8$ alkoxy and the others of $R^1$, $R^2$ and $R^3$ are hydrogen.

32. A compound according to Claim 29 wherein m is 1.

33. A compound according to Claim 29 wherein n is 3.

34. A compound according to Claim 29 which is α-[3-(4-methoxyphenyl)propyl]-4-pyridineethanol.

35. A pharmaceutical composition comprising at least one compound according to Claim 1, together with one or more non-toxic pharmaceutically acceptable carriers.

36. A pharmaceutical composition according to Claim 35 wherein said compound is selected from the group consisting of:
α-[3-(4-methoxyphenyl)propyl]-3-pyridineethanol,
α-[2-(4-methoxyphenyl)ethyl]-3-pyridineethanol,
α-[4-(4-methoxyphenyl)butyl]-3-pyridineethanol,
α-[3-(3,4-dimethoxyphenyl)propyl]-3-pyridineethanol,
α-[3-(4-methoxyphenyl)ethyl]-3-pyridinepropanol,
α-[3-(4-methoxyphenyl)propyl]-3-pyridinepropanol,
α-[3-(4-methylthiophenyl)propyl]-3-pyridineethanol,
α-(3-phenylpropyl)-3-pyridineethanol,
α-methyl-α-[3-(4-methoxyphenyl)propyl]-3-pyridineethanol, and
α-[3-(4-methoxyphenyl)propyl]-4-pyridineethanol.

37. Use of at least one compound of claims 1 to 34 for the manufacture of a medicament for treating diseases or disorders mediated by platelet-activating factor.

38. Use according to Claim 37 wherein said compound is selected from the group consisting of:
α-[3-(4-methoxyphenyl)propyl]-3-pyridineethanol,
α-[2-(4-methoxyphenyl)ethyl]-3-pyridineethanol,
α-[4-(4-methoxyphenyl)butyl]-3-pyridineethanol,
α-[3-(3,4-dimethoxyphenyl)propyl]-3-pyridineethanol,
α-[3-(4-methoxyphenyl)ethyl]-3-pyridinepropanol,
α-[3-(4-methoxyphenyl)propyl]-3-pyridinepropanol,
α-[3-(4-methylthiophenyl)propyl]-3-pyridineethanol,
α-(3-phenylpropyl)-3-pyridineethanol,
α-methyl-α-[3-(4-methoxyphenyl)propyl]-3-pyridineethanol, and
α-[3-(4-methoxyphenyl)propyl]-4-pyridineethanol.

Claims for the following Contracting State: GR

1. A compound of the formula

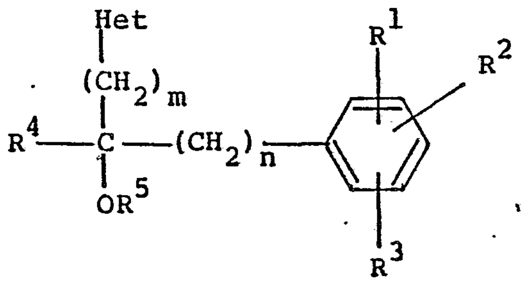

wherein $R^1$, $R^2$ and $R^3$ are independently hydrogen, $C_1$-$C_8$ alkoxy, or $C_1$-$C_8$ alkylthio;

$R^4$ is hydrogen or $C_1$-$C_8$ alkyl;

$R^5$ is hydrogen, $-\overset{O}{\overset{\|}{C}}-R^6$ or $C_1$-$C_8$ alkyl;

wherein $R^6$ is $C_1$-$C_8$ alkyl;

n and m are independently integers of from 1 to 5;

and Het is pyridyl; or a pharmaceutically acceptable acid addition salt thereof.

2. A compound according to Claim 1 wherein $R^5$ is hydrogen.

3. A compound according to Claim 2 having the formula

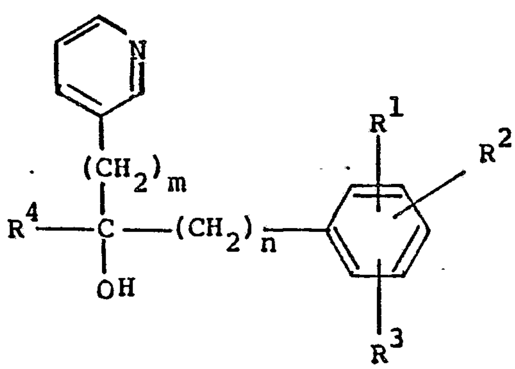

or a pharmaceutically acceptable acid addition salt thereof.

4. A compound according to Claim 3 wherein $R^4$ is hydrogen.

5. A compound according to Claim 4 having the formula

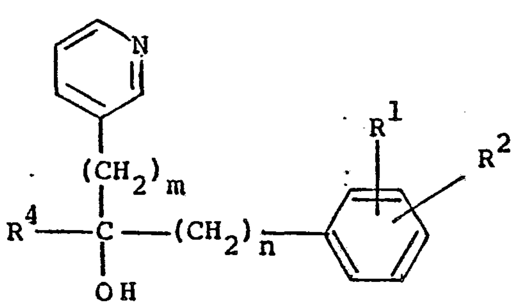

wherein one of $R^1$ and $R^2$ is $C_1$-$C_8$ alkoxy and the other of $R^1$ and $R^2$ is hydrogen or $C_1$-$C_8$ alkoxy; or a pharmaceutically acceptable acid addition salt thereof.

6. A compound according to Claim 5 wherein m is 1.

7. A compound according to Claim 6 wherein n is 2, 3, or 4.

8. A compound according to Claim 7 which is α-[3-(4-methoxyphenyl)propyl]-3-pyridineethanol.

9. A compound according to Claim 7 which is α-[2-(4-methoxyphenyl)ethyl]-3-pyridineethanol.

10. A compound according to Claim 7 which is α-[4-(4-methoxyphenyl)butyl]-3-pyridineethanol.
11. A compound according to Claim 7 which is α-[3-(3,4-dimethoxyphenyl)propyl]-3-pyridineethanol.
12. A compound according to Claim 5 wherein m is 2.
13. A compound according to Claim 12 wherein n is 2 or 3.
14. A compound according to Claim 13 which is α-[3-(4-methoxyphenyl)ethyl]-3-pyridinepropanol.
15. A compound according to Claim 13 which is α-[3-(4-methoxyphenyl)propyl]-3-pyridinepropanol.
16. A compound according to Claim 4 having the formula

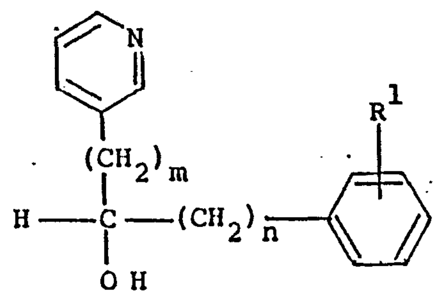

wherein $R^1$ is $C_1$-$C_8$ alkylthio; or a pharmaceutically acceptable acid addition salt thereof.
17. A compound according to Claim 16 wherein m is 1.
18. A compound according to Claim 16 wherein n is 3.
19. A compound according to Claim 16 which is α-[3-(4-methylthiophenyl)propyl]-3-pyridineethanol.
20. A compound according to Claim 4 having the formula

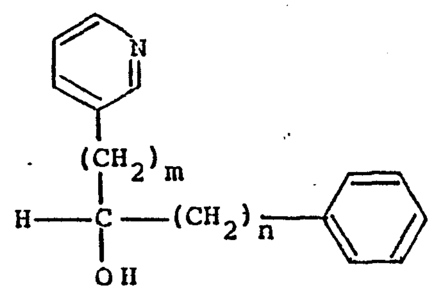

or a pharmaceutically acceptable acid addition salt thereof.
21. A compound according to Claim 20 wherein m is 1.
22. A compound according to Claim 20 wherein n is 3.
23. A compound according to Claim 20 which is α-(3-phenylpropyl)-3-pyridineethanol.
24. A compound according to Claim 3 wherein $R^4$ is $C_1$-$C_8$ alkyl.
25. A compound according to Claim 24 wherein one of $R^1$, $R^2$ and $R^3$ is $C_1$-$C_8$ alkoxy and the others of $R^1$, $R^2$ and $R^3$ are hydrogen.
26. A compound according to Claim 24 wherein m is 1.
27. A compound according to Claim 24 wherein n is 3.
28. A compound according to Claim 24 which is α-methyl-α-[3-(4-methoxyphenyl)propyl]-3-pyridineethanol.
29. A compound according to Claim 2 having the formula

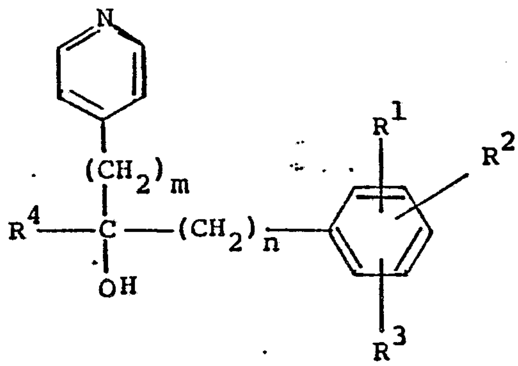

or a pharmaceutically acceptable acid addition salt thereof.

30. A compound according to Claim 29 wherein $R^4$ is hydrogen.

31. A compound according to Claim 29 wherein one of $R^1$, $R^2$ and $R^3$ is $C_1$-$C_8$ alkoxy and the others of $R^1$, $R^2$ and $R^3$ are hydrogen.

32. A compound according to Claim 29 wherein m is 1.

33. A compound according to Claim 29 wherein n is 3.

34. A compound according to Claim 29 which is α-[3-(4-methoxyphenyl)propyl]-4-pyridineethanol.

35. Use of at least one compound of Claims 1 to 34 for the manufacture of a medicament for treating diseases or disorders medicated by platelet-activating factor.

36. Use according to Claim 35 wherein said compound is selected from the group consisting of:
α-[3-(4-methoxyphenyl)propyl]-3-pyridineethanol,
α-[2-(4-methoxyphenyl)ethyl]-3-pyridineethanol,
α-[4-(4-methoxyphenyl)butyl]-3-pyridineethanol,
α-[3-(3,4-dimethoxyphenyl)propyl]-3-pyridineethanol,
α-[3-(4-methoxyphenyl)ethyl]-3-pyridinepropanol,
α-[3-(4-methoxyphenyl)propyl]-3-pyridinepropanol,
α-[3-(4-methylthiophenyl)propyl]-3-pyridineethanol,
α-(3-phenylpropyl)-3-pyridineethanol,
α-methyl-α-3-(4-methoxyphenyl)propyl]-3-pyridineethanol, and
α-[3-(4-methoxyphenyl)propyl]-4-pyridineethanol.

Claims for the following Contracting State: ES

1. A process for the producton of α-[(phenylmethoxy)methylpyridine alkanol derivatives of the general formula

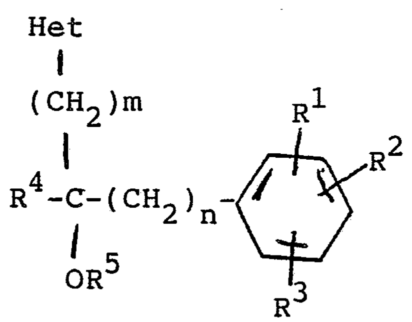

wherein $R^1$, $R^2$ and $R^3$ are independently hydrogen, $C_{1-8}$ alkoxy, or $C_{1-8}$ alkylthio,
$R^4$ is hydrogen or $C_{1-8}$ alkyl;
$R^5$ is hydrogen, $-\overset{O}{\overset{\|}{C}}-R^6$, wherein $R^6$ is $C_{1-8}$ alkyl, or $C_{1-8}$ alkyl; n and m independently are integers of from 1 to 5; and Het is pyridine;
or a pharmaceutically acceptable salt thereof;
characterized in that an aryl alkene of the general formula

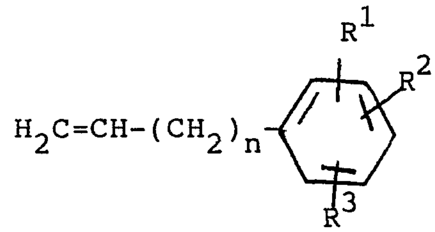

wherein $R^1$, $R^2$ and $R^3$ and n are as defined above, is treated by peroxidation with perbenzoic acid or a derivative thereof of the general formula

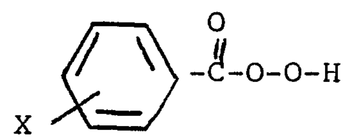

wherein X is a halogen, and the resulting epoxyalkylbenzyl derivative of the general formula

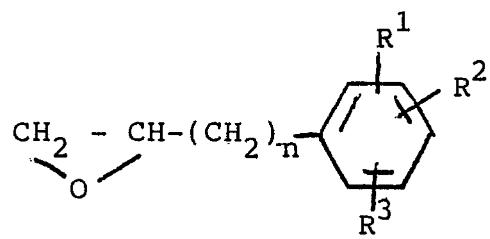

wherein $R^1$, $R^2$ and $R^3$ and n are as defined above, is reacted with a solution of bromopyridine.

2. A process for the production of $\alpha$-[(phenylmethoxy)methyl pyridine alkanol derivatives of the general formula

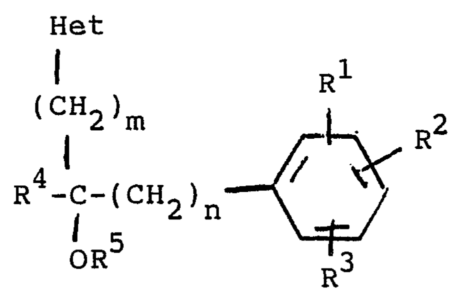

wherein $R^1$, $R^2$ and $R^3$ are independently hydrogen, $C_{1-8}$ alkoxy, or $C_{1-8}$ alkylthio;
$R^4$ is hydrogen or $C_{1-8}$ alkyl;
$R^5$ is hydrogen, $-\overset{O}{\overset{\|}{C}}-R^6$ wherein $R^6$ is $C_{1-8}$ alkyl, or $C_{1-8}$ alkyl; n and m independently are integers of from 1 to 5; and Het is pyridine; or a pharmaceutically acceptable salt thereof;
characterized in that a hydroxy alkyl benzyl compound of the general formula

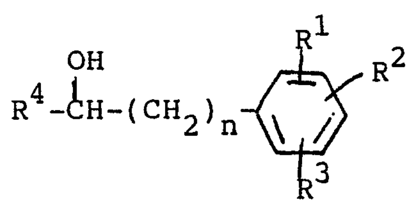

wherein $R^1$, $R^2$, $R^3$, $R^4$ and n are as defined above, is oxidized with a strong oxidizing agent, and the resulting carboxy alkyl benzyl derivative of the general formula

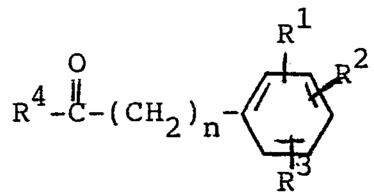

wherein $R^1$, $R^2$, $R^3$, $R^4$ and n are as defined above, is reacted with a solution of picoline or any of its isomers of the general formula

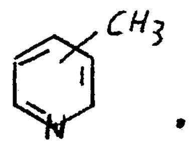

3. A process for the production of α-[phenylmethoxy)methyl pyridine alkanol derivatives of the general formula

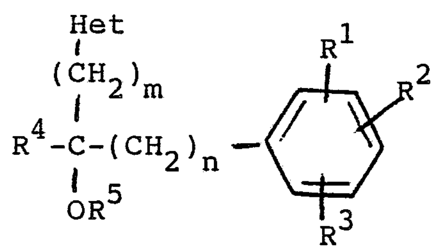

wherein $R^1$, $R^2$ and $R^3$ are independently hydrogen, $C_{1-8}$alkoxy, or $C_{1-8}$ alkylthio;
$R^4$ is hydrogen or $C_{1-8}$ alkyl;
$R^5$ is hydrogen, $-\overset{O}{\overset{\|}{C}}-R^6$ wherein $R^6$ is $C_{1-8}$ alkyl, or $C_{1-8}$ alkyl; n and m independently are integers of from 1 to 5; and Het is pyridine;
or a pharmaceutically acceptable salt thereof;
characterized in that a compound of the general formula

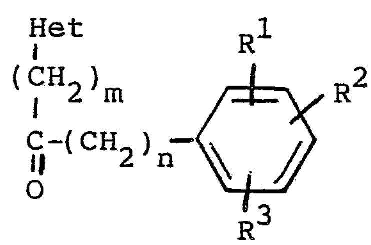

wherein $R^1$, $R^2$, $R^3$, m, n and Het are as defined above, is reduced with a compound of the general formula

$A'BH_4$

wherein A' is an alkali metal.

4. A process for the production of α-[phenylmethoxy)methyl pyridine alkanol derivatives of the general formula

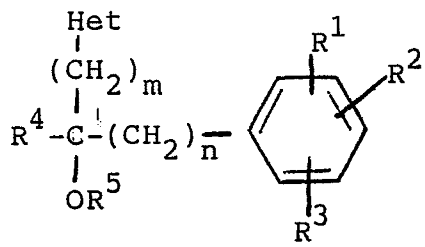

wherein $R^1$, $R_2$ and $R^3$ are independently hydrogen, $C_{1-8}$ alkoxy, or $C_{1-8}$ alkylthio; $R^4$ is hydrogen or $C_{1-8}$ alkyl;

$R^5$ is hydrogen, - $\overset{O}{\overset{\|}{C}}$ -$R^6$ wherein $R^6$ is $C_{1-8}$ alkyl, or $C_{1-8}$ alkyl; n and m independently are integers of from 1 to 5; and Het is pyridine; or a pharmaceutically acceptable salt thereof; characterized in that a compound of the formula

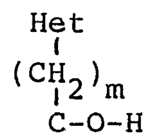

wherein m and Het are as defined above, is oxidized to the corresponding aldehyde of the general formula

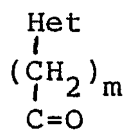

which is then reacted with a compound of the general formula

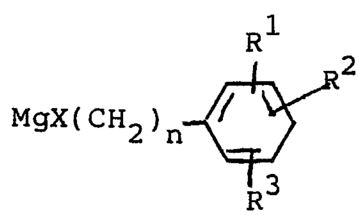

wherein $R^1$, $R^2$ and $R^3$ are as described above and X is a halogen.

5. Use of at least one compound prepared according to Claims 1 to 4 for the manufacture of a medicament for treating diseases or disorders mediated by platelet-activating factor.

6. Use according to Claim 5 wherein said compound is selected from the group consisting of:
α-[3-(4-methoxyphenyl)propyl]-3-pyridineethanol,
α-[2-(4-methoxyphenyl)ethyl]-3-pyridineethanol,
α-[4-(4-methoxyphenyl)butyl]-3-pyridineethanol,
α-[3-(3,4-dimethoxyphenyl)propyl]-3-pyridineethanol,
α-[3-(4-methoxyphenyl)ethyl]-3-pyridinepropanol,
α-[3-(4-methoxyphenyl)propyl]-3-pyridinepropanol,
α-[3-(4-methylthiophenyl)propyl]-3-pyridineethanol,
α-(3-phenylpropyl)-3-pyridineethanol,
α-methyl-α-[3-(4-methoxyphenyl)propyl]-3-pyridineethanol, and
α-[3-(4-methoxyphenyl)propyl]-4-pyridineethanol.